# EUROPEAN PATENT APPLICATION

(11) **EP 0 903 122 A2**
(43) Date of publication of application: **24.03.1999**
(21) Application number: 98307419.6
(22) Date of filing: 14.09.1998
(51) Int. Cl.: A61F 2/06

(54) **Collet-type crimping tool**

(30) Priority: 12.09.1997 US 928877
(71) Applicant: ADVANCED CARDIOVASCULAR SYSTEMS, INC., Santa Clara California 95052 (US)
(72) Inventor: Morales, Stephen A., Mountain View, California 94041 (US)
(74) Representative: McLeish, Nicholas Alistair Maxwell

(57) **Abstract**

A sterile tool for crimping a stent onto a balloon catheter is disclosed. the stent crimping tool includes two major components, a cylindrical body having external threads and a rotating collar with internal threads engaging the external threads. The collet end of the cylindrical body is split into segmented jaws that are biased to flare outward in an open state. A stent loaded onto a balloon catheter and situated inside the open-segmented jaws can undergo a crimping operation when the collar is rotated and advances toward the flared open-segmented jaws. When the collar engages the segmented jaws, the jaws are forced to converge and close onto the stent-and-catheter assembly, thereby crimping the stent onto the balloon catheter.

## Description

The present invention relates to an apparatus for loading a tubular graft, such as a stent, onto the distal end of a catheter assembly of the kind used, for example, in percutaneous transluminal coronary angioplasty (PTCA) procedures, percutaneous transluminal angioplasty (PTA) procedures, atherectomies, and the like.

In typical PTCA procedures, a guiding catheter is introduced percutaneously into the cardiovascular system of a patient through the brachial or femoral arteries and is advanced through the vasculature until the distal end of the guiding catheter is in the ostium of the aorta leading to the coronary arteries. A guide wire and a dilatation catheter having a balloon on the distal end are introduced through the guiding catheter with the guide wire sliding within the dilatation catheter. The guide wire first is advanced out of the guiding catheter into the coronary vasculature of the patient and the dilatation catheter is advanced over the previously-advanced guide wire until the dilatation balloon is positioned properly across the arterial lesion. Once in position across the lesion, a flexible and expandable balloon is inflated to a predetermined size with a radiopaque liquid at relatively high pressures, to radially compress the atherosclerotic plaque of the lesion against the inside of the artery wall and thereby dilate the lumen of the artery. The balloon then is deflated to a small profile so that the dilatation catheter can be withdrawn from the patient's vasculature and the blood flow can be resumed through the dilated artery. As should be appreciated by those skilled in the art, while the above-described procedure is typical, it is not the only method used in angioplasty.

In angioplasty procedures of the kind referenced above, restenosis of the artery may develop at or near the treatment area, which may require another angioplasty procedure, a surgical bypass operation, or some other method of repairing or strengthening the area. To reduce the likelihood of the development of restenosis and to strengthen the area, a physician can implant an intravascular prosthesis for maintaining vascular patency, commonly known as a stent, inside the artery at the treated area. The stent is transported in its delivery diameter through the vasculature. At the deployment site, the stent is expanded to a larger diameter, often by inflating the balloon portion of the catheter. The stent also may be of the self-expanding type.

Because the catheter and stent travel through the patient's vasculature, and typically through the coronary arteries, the stent must have a small delivery diameter and must be attached firmly to the catheter until the physician is ready to implant it. Thus, the stent must be loaded onto the catheter so that it does not interfere with delivery, and it must not come off the catheter until it is implanted.

In procedures where the stent is placed over the balloon portion of the catheter, it is necessary to crimp the stent onto the balloon portion to reduce its diameter and to prevent it from sliding off the catheter when the catheter is advanced through the patient's vasculature. Non-uniform crimping can result in sharp edges being formed along the now uneven surface of the crimped stent. Furthermore, non-uniform stent crimping may not achieve the desired minimal profile for the stent and catheter assembly, When the stent is not reliably crimped onto the catheter, the stent may slide off the catheter and into the patient's vasculature prematurely as a loose foreign body, possibly causing blood clots in the vasculature, including thrombosis. Therefore, it is important to ensure the proper crimping of stent onto a catheter in a uniform and reliable manner.

This crimping often is done by hand, which can be unsatisfactory due to the uneven application of force resulting in non-uniform crimps. In addition, it is difficult to judge visually when a uniform and reliable crimp has been applied.

Some self-expanding stents are difficult to load by hand onto a delivery device such as a catheter. Further, the more the stent is handled the higher is the likelihood of human error which could result in an improperly crimped stent. Accordingly, there is a need in the art for a device for reliably crimping a stent onto a catheter.

There have been attempts at devising a tool for crimping a stent onto a balloon delivery catheter. An example of such a tool comprises a series of plates having substantially flat and parallel surfaces that move in a rectilinear fashion with respect to each other. A stent-carrying catheter is disposed between these surfaces, which surfaces crimp the stent onto the outside of the catheter by reason of the relative motion and the applied pressure. The plates have multiple degrees of freedom and may have force-indicating transducers to measure and indicate the force applied to the catheter during crimping of the stent.

Another stent-loading tool design is comprised of a tubular member housing a bladder. The tubular member and bladder are constructed to hold a stent that is to be crimped onto a balloon catheter assembly. Upon placement of the stent over the balloon portion of the catheter, a valve in the loading tool is activated to inflate the bladder. The bladder compresses the stent radially inward to a reduced diameter onto the balloon portion of the catheter to achieve a snug fit. In this way, the stent is crimped onto the distal end of a balloon catheter with a minimum of human handling. The foregoing stent crimping tools are disclosed in, for example, commonly owned and assigned U.S. Patent Nos. 5,437,083 and 5,546,646 to Williams et al.

Yet another stent crimping tool is known in the art and manufactured under the tradename "BARD XT",by C.R. Bard Inc. of Boston, Massachusetts, which tool actually is a stent loader. It is constructed of a tubular body with a ball at one end that is connected to a plurality of long, thin strips which pass through the rigid tubular body. An uncrimped stent is placed over the plurality of long, thin strips, and the strips hold the stent in an expanded state. The balloon portion of a catheter is inserted into the cylindrical space formed by the plurality of strips. When the user pulls on the ball while holding the tubular body against the stent, the strips are slid from beneath the stent and the stent is transferred onto the balloon portion.

Still another conventional stent crimping tool is manufactured by the Johnson & Johnson Company and resembles a hinged nutcracker. Specifically, the tool is comprised of two hand-operated levers that are hinged at one end. The levers are designed to be gripped in the palm of a user's hand at the opposite end. A cylindrical opening holding a crimping tube is provided through the mid-portion of the tool to receive therein a stent loaded onto a balloon catheter. The crimping operation is performed when the user squeezes the levers thereby pressing the crimping tube which in turn pinches the stent onto the balloon catheter.

While the prior art devices are suitable for crimping stents onto balloon catheters, these devices suffer from problems such as non-uniform crimping forces, which result in non-uniform crimps, and thus these devices they are unsuitable for use by physicians in a catheterization lab who desire to crimp the stent into the balloon catheter.

The present invention is directed to a method and apparatus for crimping an intravascular stent onto the distal end of a catheter. The apparatus is comprised of a cylindrical body having a gripping end and a collet end, wherein the cylindrical body at the collet end transitions into a plurality of segmented jaws that are flared outward. Threads are disposed on the cylindrical body in between the collet end and the gripping end. A collar is rotatably mounted on the cylindrical body and has an internally threaded opening which engages the threads on the body. The front inside diameter of the internally threaded opening engages the plurality of segmented jaws, wherein advancing the collar along the threads translates the front inside diameter over the flared segmented jaws to converge the jaws into a closed state.

In a preferred embodiment, a groove is formed along a length of each jaw so that when the plurality of segmented jaws are in the closed state, the grooves collectively form a cylindrical cavity leading to an opening at the collet end of the cylindrical body. Thus, closing the segmented jaws onto the stent mounted to the balloon portion of the catheter when the stent and catheter are situated within the cavity crimps the stent onto the catheter.

In the preferred embodiment, a crimping apparatus according to the invention has four segmented jaws and each segmented jaw includes a generally quarter-circle, cross-sectional shape. The segmented jaws may be modified with a liner, various coatings, foam plates, or a floating head. Such modifications are aimed at gripping and holding the stent without damaging the part. Moreover, when the converged segmented jaws in the closed state are lined, the lining material ensures that the crimp which results will be characterized by a constant diameter crimp. The diameter can be adjusted by changing the thicknesses or shapes of the lining material.

In the preferred embodiment of the present invention, the tool is designed to be used in a catheterization lab to crimp intravascular stents onto balloon catheters by forcing the stent to compress from four points around its circumference onto the exterior diameter of the balloon. The balloon with the uncrimped stent mounted in the correct position thereon is placed inside the flared collet end of the cylindrical body with the collar threaded thereon halfway towards the flared collet end.

The balloon and stent are held in position by a person other than the one doing the crimping or by a table or other support. While the balloon and stent are supported, another person can twist the collar to advance it along the length of the threads, thereby forcing the segmented jaws at the collet end to converge and close down onto the stent. This closing action crimps the stent onto the balloon.

As mentioned earlier, grooves are formed along the length of each jaw so that when the jaws are in their closed state, the grooves collectively form a cylindrical cavity, the dimensions and shape of which match the crimped stent. The cylindrical cavity contains the crimped stent and the catheter balloon when the segmented jaws have fully converged. The grooves may be profiled to vary the diameter of the cavity and the contours along the length of the crimped stent. It is normal to encounter some resistance in the compression process. The stent and catheter balloon can be released from the tool by unscrewing the collar from the cylindrical body, thereby releasing the external pressure on the converged segmented jaws and allowing the jaws to open.

If the crimping process is not satisfactory, the process can be repeated for as many times as the user deems necessary. The tool and the operation thereof are extremely simple and repeatable. Indeed, by virtue of the rotating motion of the collar along the cylindrical body, it is possible to mark the tool for the precise distance the collar is advanced along the threads of the cylindrical body, for accurate crimping of the stent by the converging segmented jaws.

Embodiments of the present invention thus provide the end user with a precise and repeatable method of crimping a stent onto a balloon catheter. To achieve precision, the apparatus may be modified so as to be provided with an optional micrometer, strain gauges, or the like for tight control. In contrast, many conventional processes are unreliable and achieve inconsistent and non-uniform crimps. Further embodiments of the crimping tool of the present invention can be used with any stent that is designed to be released without a delivery system. The crimping tool may be sold with or without a stent as a component. Finally, embodiments of the present invention tool solve a common problem with conventional tools wherein crimping down to a certain diameter with precision is difficult. These and other advantages of the present invention will become more apparent from the following detailed description, when taken in conjunction with the accompanying drawings.

### BRIEF DESCRIPTION OF THE DRAWINGS

FIGURE 1 is an elevational view, partially in section, of an intravascular stent that is mounted onto a delivery balloon catheter and disposed within a vessel.

FIG. 2 is a perspective view of a preferred embodiment of a crimping tool according to the invention, showing the segmented jaws in the open state.

FIGS. 3A and 3B show a side elevational and a front view, respectively, the cylindrical body of a preferred embodiment of a crimping tool according to the invention, having a gripping end and a flared collet end, with the segmented jaws in the flared, open state.

FIGS. 4A and 4B depict a side elevational and a front view, respectively, of the collar of a crimping tool according to the invention, having internal threads, a leading edge, and a knurled exterior.

FIG. 5 is a side elevational view of an embodiment of a crimping tool according to the invention, wherein a catheter-and-stent assembly has been loaded into the collet end just prior to the crimping operation.

FIG. 6 is a side elevational view of the embodiment of a crimping tool shown in FIG. 5, wherein the crimping operation has occurred and the segmented jaws have converged onto the stent-and-catheter assembly due to advancement of the collar along the cylindrical body of the crimping tool.

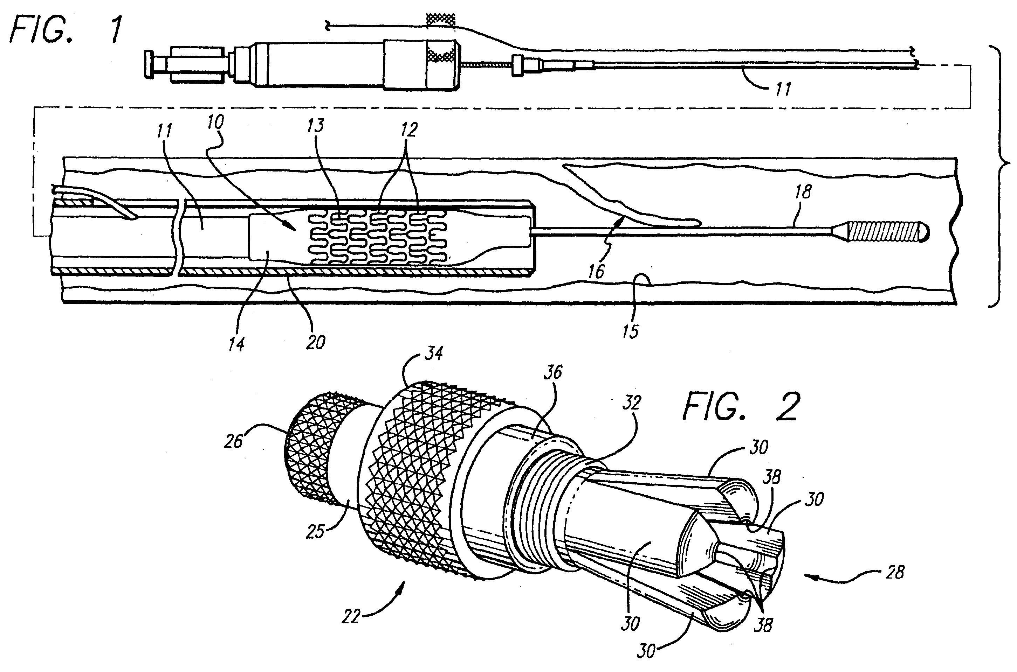

FIG. 1 illustrates an intravascular stent 10 which is mounted onto delivery catheter 11. The stent 10 generally comprises a plurality of radially expandable cylindrical elements 12 disposed generally coaxially and interconnected by the members 13 that are disposed between adjacent cylindrical elements 12. The delivery catheter 11 has an expandable portion or balloon 14 for expanding stent 10 within artery 15 or other vessel. An artery 15, as shown in FIG. 1, has a dissected lining 16, which has occluded a portion of the arterial passageway.

The delivery catheter 11, onto which the stent 10 is mounted, essentially can be the same as the type of balloon dilatation catheter that conventially is used for angioplasty procedures. The balloon 14 may be formed of suitable materials such as polyethylene, polyvinyl chloride, polyethylene terephthalate and other like polymers. In order for the stent 10 to remain in place on the balloon 14 during delivery to the site of the damage within the artery 15, the stent 10 is compressed onto the balloon 14. This compressing step is known as crimping

An optional retractable protective delivery sleeve or sheath 20 may be provided to further ensure that the stent 10 stays in place on the balloon 14 of the delivery catheter 11 and to prevent abrasion of the body lumen by the open surface of the stent 10 during delivery to the desired arterial location. Other means for securing the stent 10 onto the balloon 14 also may be used, such as providing collars or ridges on the ends of the working portion, i.e., the cylindrical portion, of the balloon 14.

The catheter-and-stent assembly can be introduced into the vasculature of the patient through processes that are known in the art, generally immediately following PTCA, PTA, or atherectomy procedures. Briefly, a guide wire 18 is disposed across the treated arterial section, referred to as the target area 16, and the catheter-and-stent assembly is advanced over the guide wire 18 within the artery 15 until the stent 10 is directly under the target area 16. Prior to inflation of the balloon 14, the delivery sleeve 20 is retracted to expose the stent 10. The balloon 14 of the delivery catheter 11 then is expanded using an inflation fluid. Expansion of the balloon 14 in turn expands the stent 10 into contact with the artery 15. Next, the balloon 14 is deflated and the catheter 11 is withdrawn, leaving the stent 10 to support the target area 16. As mentioned above, in order to ensure proper seating of the stent 10 onto the balloon 14, and to ensure proper deployment of the stent 10 at the target area 16 within the artery 15, the stent crimping procedure is critical.

In order to implant the stent 10 in a vessel, it first is mounted onto the inflatable balloon 14 on the distal extremity of the delivery catheter 11. The stent 10 is crimped down onto the balloon 14 to insure a low profile. The present invention encompasses this crimping procedure.

FIG. 2 provides a perspective view of a preferred embodiment of a stent crimping tool 22 according to the invention. In the preferred embodiment as shown, the stent crimping tool 22 is comprised of a cylindrical body 25 having a gripping end 26 which is opposed to a collet end 28. The collet end 28 is comprised of a plurality of flared, open-segmented jaws 30 which are formed by splitting the collet end 28 into discrete branches.

The cylindrical body 25 further includes external threads 32 disposed in between the gripping end 26 and the collet end 28. The stent crimping tool 22 further includes a rotating cylindrical collar 34 having internal threads that engage the external threads 32 of the cylindrical body 25. The cylindrical collar 34 also includes a leading edge 36 that is designed to engage the segmented jaws 30 as the collar 34 is advanced forward towards the collet end 28. As this engagement progresses, the leading edge 36 forces the segmented jaws 30 from the flared, open state into a closed state, in which all of the segmented jaws 30 converge towards a theoretical axial center line of the cylindrical body 25. FIG. 2 further illustrates an optional groove 38, preferably formed into each segmented jaw 30, that extends the length of each jaw 30.

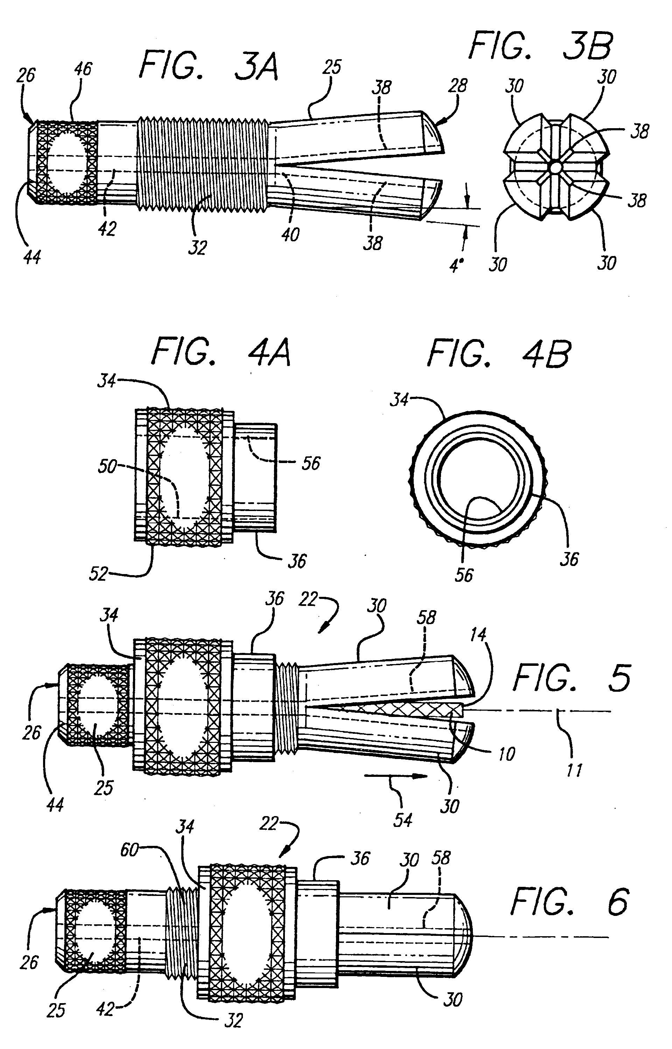

As is best seen in FIGS. 3A and 3B, which respectively provide a side elevational view and a front view of the cylindrical body 25 of a crimping tool according to the preferred embodiment of the invention, the groove 38 transitions at a confluence point 40 into a bore 42 which extends the length of the cylindrical body 25. The gripping end 26 optionally may be knurled 46 to provide a better gripping surface. Other finishing methods known in the art can be used to enhance the friction at this point as well.

The cylindrical body 25 preferably is approximately 9.53 centimeters (3.75 inches) long, however, this length can vary depending upon the application. The gripping end 26 is characterized by a chamfered edge 44 and, as mentioned above, a knurled surface 46 can be provided just beyond the chamfered edge 44 to allow a better grip. Just beyond the knurled portion 46 are external threads 32, which in the preferred embodiment, are a 5 degree coarse thread. The external threads 32 wind to approximately 6.35 centimeters (2.5 inches) up the shaft of the cylindrical body 25. Then the profile of the cylindrical body 25 flares out from its center axis, preferably at a 4 degree angle, and transitions into the collet end 28. The collet end 28 not only is flared out but also is split down the shaft about 6.35 centimeters (2.5 inches) where the threads begin, approximately coinciding with the confluence point 40. The split preferably is a 4 degree taper that begins at the 6.35 centimeters (2.5 inches) mark from the gripping end 26 and expands at 4 degrees (relative to the center axis) to the collet end 28, creating an approximate 0.48 centimeter (0.189 inch) gap between the ends of the cylindrical body 25.

In this embodiment, the collet end 28 is split preferably into four discrete segmented jaws 30 and each segmented jaw 30 flares outward at an approximate 4 degree angle relative to the axial center line of cylindrical body 25. The segmented jaws 30 preferably are formed from four quarter-tube sections that are defined by a small inner radius of 1.78 millimeters (0.007 inch). In the flared, open state, the outside diameter of the open-segmented jaws 30 is approximately 3.06 centimeters (1.205 inches) contrasted with the preferably 2.54 centimeter (1 inch) diameter of the unflared section of the cylindrical body 25. The groove 38 formed in each segmented jaw 30 is best seen in the front view of the cylindrical body 25 in FIG. 3B.

FIGS. 4A and 4B provide a side elevational view and a front view, respectively, of the cylindrical collar 34 that is rotatably mounted to the cylindrical body 25. In the preferred embodiment shown in FIGS. 4A and 4B, the cylindrical collar 34 has a generally cylindrical shape with a first diameter, and has a leading edge 36 with a smaller second diameter. Therefore, as seen in the side elevational view of FIG. 4A, the cylindrical collar 34 preferably has a step-down diameter. The cylindrical collar 34 thus is a two-stage cylinder that, in the preferred embodiment, is approximately 3.81 centimeters (1.5 inches) in length with a maximum diameter of 5.08 centimeters (2 inches). The inner diameter is a constant at 2.54 centimeters (1 inch), and is tapped with a 5 degree coarse internal thread 50 to match the external threads 32 on the cylindrical body 25. The large diameter section of the cylindrical collar 34 preferably is 2.54 centimeters (1 inch) in length and has a 5.08 centimeter (2 inch) diameter, and further is chamfered on both edges. This section also has a knurled surface 52 to facilitate a better grip on the part.

The leading edge 36 has a 1.27 centimeter (0.5 inch) length and is cut down in diameter to preferably 3.81 centimeters (1.5 inch). The leading edge 36 is significant in forcing the flared outward segmented jaws 30 from the open state into the closed state, in which each segmented jaw 30 converges toward the axial center line of cylindrical body 25.

FIGS. 5 and 6 are side elevational views of a preferred embodiment of a stent crimping tool 22 according to the invention. In FIG. 5, the segmented jaws 30 are flared open to receive the balloon catheter 11 with the stent 10 loaded onto the balloon 14. The stent-and-balloon catheter assembly are shown in FIG. 5 as situated within a space or cavity 58 that is formed by the segmented jaws 30 in the flared, open state.

As was mentioned above, a stent crimping tool 22 according to the invention is designed to be used in a catheterization lab to crimp stents onto the balloon portions of catheters by compressing the stent onto the balloon, preferably at four points around the outer circumference of the catheter. Therefore, as seen in FIG. 5, the balloon 14, with the uncrimped stent mounted in the correct position is placed inside the collet end 28 of the cylindrical body 25. At this stage, the cylindrical collar 34 already has been threaded halfway towards the collet end 28, preferably 3.81 centimeters (1.5 inches) as measured from the back edge of the gripping end 26. The stent-and-catheter assembly can be advanced in through either method until it is aligned in the collet segments.

In the catheterization lab, ideally the stent-and-catheter assembly is held in position by a person other than the one using the crimping tool, or by a table, or other support known in the art. While the stent-and-catheter assembly is supported, the crimping tool user can twist the cylindrical collar 34, thereby advancing the collar 34 along the cylindrical body 25 in the direction of the arrow 54.

By this motion, the leading edge 36 is translated into engagement with the segmented jaws 30. The preferably smooth inside diameter 56 of the cylindrical collar 34 slides over the flared, open-segmented jaws 30. As the cylindrical collar 34 is advanced further in the direction of the arrow 54, the inside diameter 56 engages and forces the segmented jaws 30 from the open state to converge toward an axial center line of the cylindrical body 25 into a closed state. The convergence of the segmented jaws 30 into the stent-and-catheter assembly compresses the stent 10 onto the balloon 14.

The cavity 58 is formed when the segmented jaws 30 converge and completely engage the stent-and-catheter assembly. The cavity 58 is partially formed by the collective convergence of the grooves 38. FIG. 6 shows this condition in which the segmented jaws 30 have assumed the closed state and the cylindrical collar 34 has been advanced fully forward on the cylindrical body 25. As seen in FIG. 6, the bore 42 is needed to accommodate longer catheters in which the balloon sits further back.

The stent-and-catheter assembly can be released by unscrewing the cylindrical collar 34 from the cylindrical body 25. This action disengages the leading edge 36 from contact with the segmented jaws 30 which preferably are biased to flare outward. The segmented jaws 30 then return to the flared, open state thus releasing the stent-and-catheter assembly and permitting removal thereof.

If the crimp is not satisfactory, the crimping process can be repeated for as many times as necessary to achieve a tight, uniform crimp. As seen here, the operation of the stent crimping tool 22 is simple, repearable, and can be controlled precisely. In fact, with the rotating motion of the cylindrical collar 34, it is possible to mark the cylindrical body 25 to indicate the amount of forward advancement translating to the amount of convergence in the segmented jaws 30, thereby accurately crimping the stent 10. To ensure accuracy, and as illustrated in FIG. 6, an optional indicator mark 60 can be provided to help the user control the amount of advancement of the cylindrical collar 34 and the associated amount of convergence of the segmented jaws 30 during the crimping operation.

As will be appreciated by those skilled in the art, a crimping tool 22 according to the present invention is designed both for single use applications in a catheterization lab by a physician, or for multiple use applications in a sterile environment in a high-volume manufacturing facility. In the manufacturing facility where sterile conditions exist, the stent crimping tool 22 can be used repeatedly to crimp stents onto balloon catheters until the mechanism wears out. Thus, repeated uses of the present invention are contemplated for controlled, sterile environments although single use applications are required when used by catheterization lab personnel.

Embodiments of a stent crimping tool according to the invention can be used with any stent that is released without a delivery system. The crimping tool also may be sold alone, because its design is robust enough to undergo many uses. The crimping tool 22 can be used to crimp any expandable stent onto any catheter, and particularly is suitable for stents implanted in the coronary arteries, arteries, veins, and other body lumens. The tool also is suitable to crimp saphenous vein grafts.

In a preferred embodiment, all of the parts of the stent crimping tool 22 are made from machined polymers. Alternatively, the present invention also is well suited to be made from surgical steel, aluminum, or other metals, such that it can be used and reused.

In an alternative embodiment, the grooves of the segmented jaws and the jaws can be coated with rubber or other resilient materials. In addition, in other alternative emboditments, the grooves and/or the segmented jaws can include floating heads, foam plates, or a lining, in order to effect a desired outside diameter or a specific profile for the stent. For the same effect, a lining also may be used to cover the stent-and-catheter prior to crimping.

The split in the cylindrical body 25 at the collet end 28 can be achieved by using a band saw or rotating blade-type tool, wherein the natural reaction of the polymer base material is to bias the segmented jaws outward into a flared configuration. It also is possible, through processes known in the art, to heat treat the polymer to create the outward bias of the flared segmented jaws.

Other modifications can be made to the present invention without departing from the scope thereof. The specific dimensions, procedural steps, and materials of construction are provided as examples, and substitutes are readily contemplated which do not depart from the invention.

## Claims

1. A tool (22) for crimping a stent onto a catheter (11), comprising:
a cylindrical body (25) having a gripping end (26) and a collet end (28), wherein the cylindrical body (25) at the collet end (28) transitions into a plurality of segmented jaws (30) that are flared outward;
external threads (32) disposed on the cylindrical body (25) in between the collet end (28) and the gripping end (26);
a collar (34) rotatably mounted on the cylindrical body (25) wherein an internal threaded opening engages the external threads (32) on the body and a front inside diameter of the internal threaded opening engages the plurality of segmented jaws (30);
wherein advancing the collar (34) along the threads translates the front inside diameter over the segmented jaws (30) to converge the segmented jaws (30) into a closed state; and
a groove (38) formed along a length of each segmented jaw (30), wherein when the plurality of segmented jaws (30) are in the closed state, the grooves (38) collectively form a cylindrical cavity (58) leading to an opening at the collet end (28) of the cylindrical body (25);
whereby closing the segmented jaws (30) onto the stent (10) mounted on the catheter (11), when the stent and catheter are situated within the cavity, crimps the stent onto the catheter.

2. The tool (22) according to claim 1, wherein the tool includes four segmented jaws (30) and each segmented jaw (30) includes a generally quarter-tubular wall cross-sectional shape.

3. The tool according to claim 1, wherein the groove (38) formed along each segmented jaw is lined.

4. The tool according to claim 1, wherein the collar (34) includes an annular cross-sectional shape.

5. The tool according to claim 1, wherein the collar (34) includes a segment that has a smooth inside diameter.

6. The tool (22) according to claim 1, wherein the cylindrical body (25) includes a metallic material.

7. The tool (22) according to claim 1, wherein the collet end (28) is rounded.

8. The tool according to claim 1, wherein the segmented jaws (30) include a coating of material on at least a portion thereof.

9. The tool according to claim 1, wherein the apparatus includes a polymer.

10. A tool (22) for crimping a stent onto a catheter, comprising:
a cylindrical body (25) having a gripping end (26) and a collet end (28), wherein the collet end (26) includes a plurality of segmented jaws (30) having a flared, open state and a converged closed state;
an opening in the collet end leading to a cavity (58) formed collectively by the plurality of segmented jaws in the converged closed state;
external threads (32) disposed on the cylindrical body (25) ;
a collar (34) having an internal opening with internal threads, wherein the collar (34) is rotatably disposed on the body with the internal threads, engaging the external threads on the body;
wherein the segmented jaws (30) are biased to flare outward toward the open state and the collar (34) overcomes the bias when it is advanced along the external threads (32) to partially engage the segmented jaws (30) and to converge the segmented jaws (30) together into the closed state; and
whereby converging the segmented jaws (30) on the stent positioned on the catheter (11) located within the cavity (58) crimps the stent (10) onto the catheter (11).

11. The tool according to claim 10, wherein the cavity has a cylindrical shape, and the cavity extends along an axial length of the cylindrical body.

12. The tool according to claim 10, wherein at least one of the segmented jaws includes a coating on at least a portion thereof.

13. The tool according to claim 10, wherein the collet end includes a tubular cross-sectional shape.

14. The tool according to claim 10, wherein each segmented jaw in the flared open state defines at least a four degree angle from a longitudinal axis of the cylindrical body.

15. The tool according to claim 10, wherein the collar includes a cylindrical shape having an overall diameter with a step-down profile defining a leading edge having a diameter smaller than the overall diameter of the collar, and wherein the leading edge is proximal to and engages the segmented jaws to converge the segmented jaws into the closed state as the collar is advanced along the external threads of the body.

16. The tool according to claim 10, wherein the cavity is configured to receive a balloon portion (14) of a catheter (11).

17. A method for crimping a stent onto a catheter, comprising the steps of:
providing a cylindrical body (25) having a gripping end (26) and a collet end (28), wherein the collet end (28) includes a plurality of segmented jaws (30) having a flared, open state and a converged closed state;
providing an opening in the collet end leading to a cavity (58) formed collectively by the plurality of segmented jaws (30) in the converged closed state;
adding external threads (32) onto the cylindrical body (25);
providing a collar (34) having an internal opening with internal threads;
advancing the collar (34) onto the body, wherein the internal threads engage the external threads (32) on the body;
biasing the segmented jaws (30) to flare outward toward the open state;
positioning the stent (10) coaxially and overlying the catheter (11);
inserting the stent (10) and catheter (11) into the cavity (58); and
rotating the collar (34) along the external threads (32) to partially engage the segmented jaws (30) to overcome the bias in order to converge the segmented jaws together into the closed state;
whereby converging the segmented jaws (30) on the stent (10) crimps the stent (10) onto the catheter (11).

18. The method according to claim 17, wherein the cavity (58) has a cylindrical shape.

19. The method according to claim 17, wherein the step of biasing the segmented jaws (30) includes deforming each segmented jaw (30) outward.

20. The method according to claim 17, wherein the step of providing the cylindrical body (25) having a gripping end (26) and the collet end (28) further comprises the step of splitting the cylindrical body (25) at the collet end (28) to form the segmented jaws (30).
